# EUROPEAN PATENT SPECIFICATION

(11) **EP 0 398 394 B1**
(45) Date of publication and mention of the grant of the patent: **06.10.1993**
(21) Application number: 90113244.9
(22) Date of filing: 08.11.1984
(51) Int. Cl.: A61M 5/145, B67D 1/00

(54) **Reservoir assembly for removable engagement with a motor drive**
Behälteraufbau für die auswechselbare Verbindung mit einem Motorantrieb
Ensemble-réservoir destiné à être connecté de façon détachable à une commande par moteur

(30) Priority: 15.11.1983 US 551851
(43) Date of publication of application: 22.11.1990
(62) Divisional of application: 84904294.0
(73) Proprietor: KAMEN, Dean L., Bedford, NH 03101 (US)
(72) Inventor: KAMEN, Dean L., Bedford, NH 03101 (US)
(74) Representative: Kraus, Walter, Dr.

(56) References cited:
- GB-A- 2 015 883
- US-A- 3 858 581

## Description

The invention relates to a reservoir assembly for removable engagement with a motor drive, comprising:
(a) a reservoir; and
(b) a piston having (i) a fluid contacting head portion located within the reservoir and (ii) a drive body for engagement with a motor drive and having an aperture therein.

This reservoir assembly is particularly suited for medical infusion pumps and more particularly pumps capable of home use by ambulatory patients and pumps having built in controls for sustained or periodic infusions over a substantial time.

Such a reservoir assembly is defined in the preamble of claim 1 and known from US-A-3 858 581.

Much interest has arisen in recent years in the development of medical infusion pumps capable of relatively continuous operation and also in the development of infusion pumps having the ability to administer precisely controlled volumes of fluid at preselected intervals. Unlike the case with a parenteral fluid administration apparatus, which typically operates by gravity flow from a relatively large reservoir of fluid, medication infusion generally involves administration of small dosages so as to achieve a prescribed optimum level of medication in the bloodstream of the patient. Due to differences in the rates of metabolism of, or elimination of, various medicines, the attainment of precise bloodstream medication levels is usually achieved by the periodic administration of a dosage of medication, where determination of the precise dosage and the correct interval between doses will depend on the particular medication involved as well as criteria peculiar to the patient. Such repeated administration of medicines can be difficult to achieve because of erratic communications with, or supervening demands, on hospital nursing staff; moreover repeatedly giving a patient injections can be traumatic. Thus emphasis has grown on developing versatile medical infusion pumps to automatically administer a sustained sequence or rate of infusions. Pumps of several types are known in the prior art. One species of pump involves rather massive mechanical elements for the pump and dosage setting device so as to give the very precisely controlled sequence of high pressure infusions necessary for radiography. Such devices are disclosed in US-A-3 701 345 and US-A-3 812 843 among others.

Furthermore, US-A-3 858 581 discloses a reservoir assembly for removable engagement with a motor drive, comprising a reservoir, and a piston having a fluid contacting head portion located within the reservoir and a drive body for engagement with a motor drive. In this reservoir assembly the reservoir and the piston are those of a usually manipulatable syringe having a syringe chamber as reservoir and a plunger as piston, wherein the linear displacement of the plunger dictates the amount of medication which is dispensed from the syringe chamber through a tubing to the patient.

The motor drive of this reservoir assembly according to US-A-3 858 581 is constructed such that the syringe can be releasably clamped to a top plate of the housing of that motor drive and that the rearward end of the plunger can be inserted in a cap-like upstanding pushing head which has a threaded member driven for linear motion by a lead screw. The lead screw is rotated by a motor and connected thereto through a shaft coupling having a clutch.

In such reservoir assembly of US-A-3 858 581 there is provided a safety device feature which disconnects the drive between the motor and lead screw in the event of successive buildup of pressure which is transmitted in a reverse direction through the tube connected with the patient into the syringe chamber and against the syringe plunger. This buildup of resistance pressure will be transmitted against the pushing head and thus is manifested as a force tending to push lead screw in a direction away from the motor. In response to this force, the clutch of the shaft coupling will break its driving connection between its driving and driven elements and thus result in discontinuation of the driving connection between the motor and the lead screw.

However, the reservoir assembly according to US-A-3 858 581 does not contain any safety feature which prevents the removal of the piston or the refilling of the reservoir, i.e. of the syringe chamber since any syringe used in this reservoir assembly is a usually manipulatable syringe and can be unclamped from the motor drive and otherwise used whenever the user desires that.

Such safety feature however would be of great importance in a device of such nature which is intended for use in administering infusions over extended periods of time thus entailing some likelihood of contamination, or incubation of bacterial contaminants. For this reason, it is necessary to guard against misuse, particularly the possiblity that reservoir units may be removed and refilled with medication. Such refilling is poor medical practice, and would pose a substantial risk of introducing pyrogenic, infectious or otherwise toxic contaminants into the infusion fluid.

The present invention overcomes the foregoing and other limitations and provides a reservoir assembly of the type mentioned in the beginning which is characterized by a severable stem removably attaching the fluid contacting head portion to the drive body, protruding from the fluid contacting head portion through the aperture in the drive body sufficiently far to prevent the drive body from being engaged with the motor drive without the stem first having been severed, so that when the piston is placed in engagement with the motor drive the head portion is detached from the body and retraction of the head portion and refilling of the reservoir are prevented.

This reservoir assembly of the present invention can be used for a pump including a reservoir, piston and drive body, in which the drive body may be internally threaded for a substantial length. A relatively short drive screw, which may be motor driven, engages the drive body to propel the piston in the reservoir. By using a piston drive body in which the skirt is omitted on one side, it is possible to place the motor and drive screw substantially within the extended piston member body, realizing a substantial reduction in space requirements. Additionally, such a structure requires no drive body journals, and dispenses with the cumbersome rack or leadscrew characteristic of prior art devices. There may result a pump structure with minimal mass, minimal frictional losses or mechanical inefficiency, and no mechanical backlash. The reservoir, piston, and drive body constitutes an assembly that can be removed from the pump. In one preferred embodiment, interlocking detents on the piston and reservoir walls may be used to prevent withdrawal or re-use of the reservoir assembly or components after use.

The shearable stem, which may be integrally formed with the piston face, holds the pistion and drive body together for setting up or bleeding the pump. The stem then is sheared off by twisting so that the piston face floats free, preventing re-use of the reservoir assembly.

The invention is well adapted for use with battery powered drive, and microprocessor chip control programming means, for dependable use with small power requirements in hospital bedside or ambulatory outpatient settings.

One embodiment of the reservoir assembly is characterized in that the stem extends along a central longitudinal piston axis and blocks the motor drive from engaging an internal surface of the piston.

Another embodiment of the reservoir assembly is characterized in that the stem is of sufficient strength to transmit push and pull forces for filling and bleeding the assembly, but has a weak portion which may be severed by twisting, so as to detach the head portion from the body.

Yet another embodiment of the reservoir assembly is characterized in that the stem is integrally formed with the piston head portion.

A still further embodiment of the reservoir assembly is characterized in that the drive body has affixed thereto a piston follower, threaded along its interior for a length greater than the desired total piston displacement.

These and other features of the invention will become apparent by reference to the drawings.

### Brief Description of the Drawings

Figure 1 A shows a perspective view of a programmable microprocessor-controlled pump which can use a reservoir assembly of the invention.

Figure 1B shows a perspective view of a pump similar to that of Figure 1A.

Figure 2 shows details of a disposable reservoir, in accordance with the present invention, with the piston and drive body withdrawn.

Figures 2A, 2B and 2C show various embodiments of a reservoir safety retaining feature utilizing a lip, barb or groove.

Figure 3 shows a side view of the piston and drive body, showing the placement of the motor and drivescrew within its contours.

Figure 3 A shows a cross-sectional view of the drive body and safety barb.

Figure 3B shows a cross-sectional view of the drive body and the breakaway safety stem.

Figure 4 shows a perspective view of the drive body, skirt profile and drive engagement threads.

Figures 5A, 5B and 5C show different embodiments of the piston adapted to provide a fluid seal.

Figure 6 shows a large volume pump adapted for sustained bedside infusion.

Figure 7 shows a section along line 7-7 of Figure 6, showing the motor and drive.

Figure 8 shows the pump of Figure 6 having the retaining slide cover.

Figure 9 shows the large volume pump of Figure 6 including the housing adapted to house a keyboard, control circuitry and power supply.

### Detailed Description of the Invention

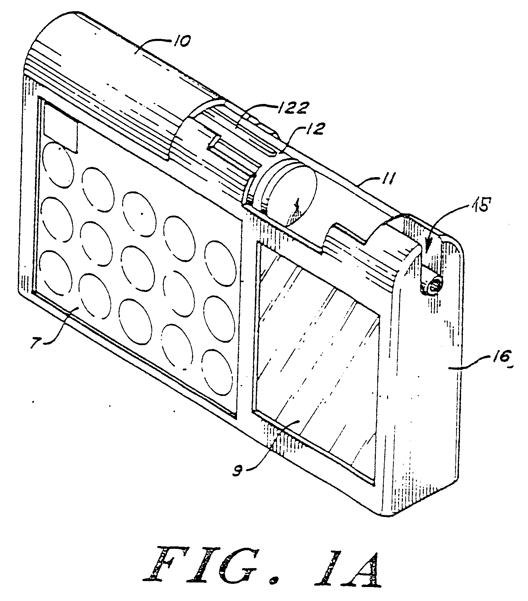

Figure 1A shows a perspective view of a complete infusion pump , in which a housing 16, containing a power supply and microprocessor control circuitry, accommodates a motorized drive unit and a disposable piston/reservoir assembly in its upper portion. A keyboard 7, preferably of an impermeable, e.g. membrane-type, construction is used to enter program information to set a schedule of amounts and timing of medication doses. A display 9 is used to display the instrument mode, to give instructions for data entry, and to signal certain operator correctable steps and alarm states. In the upper portion of the housing is mounted a reservoir 11 with a centrally disposed elongated drive body 12. The reservoir 11 is secured at one end by a gentle snap fit within opposing portions of a yoke 15 formed by the housing body. (A single side of yoke 15 is shown in this figure.) At the other end of the reservoir is an axially-sliding cover portion 10 of the housing which overlies the end of the reservoir 11 and bears against the longitudinal portion of drive body 12 to align it. To minimize friction against the drive body 12 as it slides within the reservoir 11, there is provided a protruding longitudinal ridge 122 comprising the area of contact between the piston member and the reservoir 11 and sliding cover 10.

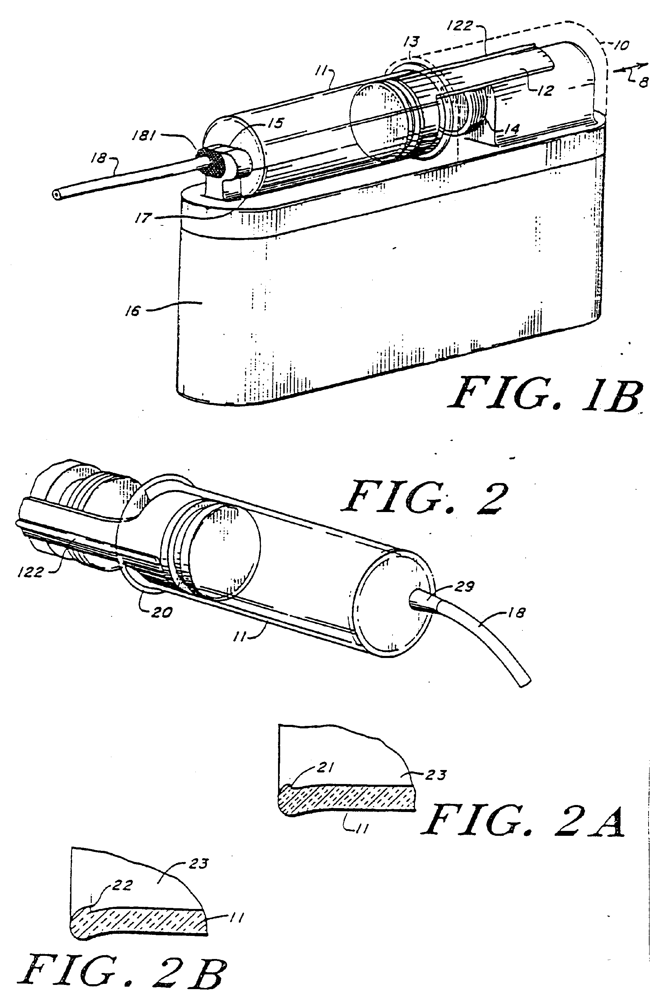

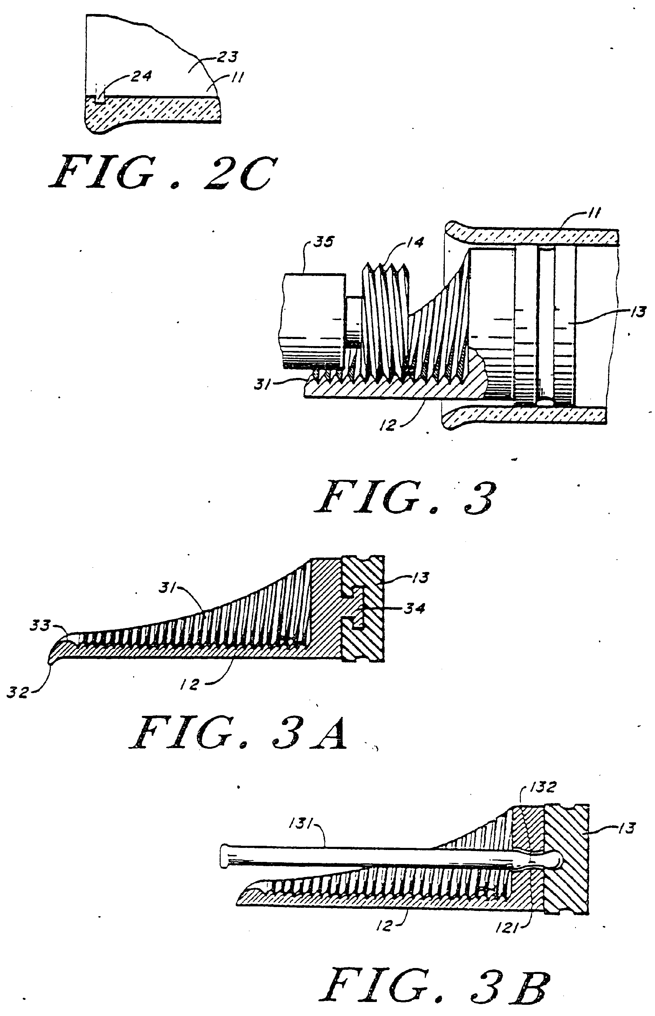

Figure 1B shows a perspective view of a complete infusion unit similar to the embodiment of Figure 1A and viewed from the opposite side. A reservoir 11, which may be graduated along its surface, fits into a recess 17 in the unit's housing 16 and is retained by the yoke 15 which varies only slightly from the yoke shown in Figure 1A. The reservoir 11 is substantially in the form of a hollow cylinder, and yoke 15 is positioned so as to retain the reservoir in axial alignment. It has been found that the use of the split yoke 15 permits convenient insertion of the reservoir 11 into recess 17. This yoke is located to position the outlet nipple, shown at 29 in Figure 2, leaving space for the attachment of a connector 181 and infusion tube 18, both of which are of a conventional design. Also shown in Figure 1B are the drive body 12, a piston 13 with a substantially circular head for hermetically displacing the fluid in the reservoir 11 when driven by the rotating screw drive 14 on the shaft of the drive motor (not visible). As shown, the housing 16 removably holds the reservoir 11 and drive body 12 as a unit. The circular piston head 13 displaces fluid in the reservoir upon axial motion of the drive body. The rearward portion of the piston member is shaped like a longitudinal segment of cylinder as shown and is preferably internally threaded so that it may be inserted into a position of engagement with drive screw 14. Drive screw 14 is a finely threaded screw gear, of a diameter to mesh with the internal threads of the drive body 12.

In the embodiment shown, all of the mechanical elements of the pump and drive are housed in the top portion of the housing 16 and the remainder of housing 16 serves to hold a battery or rechargeable power pack and a microprocessor control and rate selection means for the device. A cover 10, shown in phantom, slides in the direction of axis 8 so as to hold the drive body 12 in engagement with the drive screw after the reservoir assembly is inserted in the housing. Alternatively, the cover 10 may be an integral fixed part of the housing 16, and provided with a slot on the side thereof and longitudinally disposed along axis 8; the slot would be sufficient in size to permit insertion of the drive body 12 when the piston-reservoir assembly is first loaded into the motor assembly. After the insertion of the drive body 12 into the slot, the drive body 12 is rotated about axis 8 until the drive body 12 assumes operating position underneath the cover, where the drive body 12 may be retained by one or more nubs protruding toward axis 8 from the inside of the cover near the slot. In this manner the cover holds the piston in engagement with the drive screw.

Figure 2 shows the reservoir 11, with its outlet nipple 29. The outlet nipple may be of any conventional shape such as a taper or bayonet lock, for attachment of a sterile connector fitting 181 and infusion tube 18. When used to center the reservoir with respect to a retaining stop as shown at 15 in Figure 1B the nipple must be of sufficient length so that the stop 15 does not interfere with attachment of the connector fitting.

At the other end of the reservoir is the rear edge 20 of the circular opening constituting the end of the reservoir chamber. The present device is intended for use in administering infusions over extended periods of time thus entailing some likelihood of contamination, or incubation of bacterial contaminants. For this reason, it is necessary to guard against misuse, particularly the possibility that reservoir units may be removed and refilled with medication. Such refilling is poor medical practice, and would pose a substantial risk of introducing pyrogenic, infectious or otherwise toxic contaminants into the infusion fluid.

Accordingly a safety feature has been incorporated to prevent withdrawal of the piston and refilling of the reservoir. This safety feature includes a modification to the inner surface contour of the reservoir adjacent to the rear edge 20 of the reservoir, together with a mating modification to the distal end of the drive body. Such modifications to the reservoir assembly are shown in cross-section in Figures 2A, 2B, and 2C, and may include a lip 21, a barb 22, or a groove 24 located to engage the drive body and prevent its withdrawal after use. The drive body has a corresponding barb 32 shown in Figure 3A projecting radially outward from its distal end which engages the mating portion of the reservoir wall for causing the pistion to be locked into the reservoir upon full insertion. The drive body also has a rounded shoulder 33 preferably extending along each edge of the drive body, the prevent gripping the body which could defeat the safety lock. It has been found that the lip 21 of barb 22 of the reservoir need not protrude more than a few thousandths of an inch above the interior face 23 of the reservoir chamber to be effective, and thus need introduce no unreasonable frictional drag upon the drive body. Frictional drag may be altogether eliminated by use of the groove 24 and barb 32 embodiment of the safety interlock.

In Figure 3B, piston 13 is attached to a safety stem 131, which may be integrally formed with piston 13, extending through aperture 121 in piston body 12, and holding piston 13 to drive body 12. Safety stem 131 is capable of transmitting force in either direction along its length, and accordingly to set up and bleed a reservoir it is only necessary to push or pull on stem 131 as for a conventional syringe. However, with stem 131 intact, it is not possible to place the reservoir into the housing 16 or into engagement with the motor drive gear 14. Accordingly, to mount the assembly for operation, the stem is twisted, breaking off at a narrowed stress point 132, thus removing the obstacle to motor engagement. With the stem thus removed, the piston 13 is no longer attached to the drive body 12. It will advance when driven by the drive body, but will remain within the reservoir and will not retract when drive body 12 is withdrawn. Piston 13 may contain a polygonal recess fitted over a corresponding polygonal protrusion of drive body 12 to prevent piston 13 from turning as the stem is twisted to break it away, thus facilitating the breaking away of the safety stem.

Figure 3A shows a longitudinal cross-sectin of one embodiment of the drive body 12, in which 34 is a nub integrally formed with drive body 12 for retaining the piston 13. Internal screw threads 31 extend the length of the inside face of the drive body back from the front face, culminating in a rond shoulder, 33 visible at the distal edge. On the exterior face of the drive body 12 precisely at the distal end thereof is a slight protrusion 32 which engages the corresponding lip 21, barb 22 or groove 24 shown at Figures 2A, 2B, or 2C when the piston has been fully displaced into the reservoir 11, thus preventing removal of the piston or refilling of the reservoir. In this regard the shoulder 33 further discourages gripping of the piston, to safeguard against removal.

Referring now to Figure 3, there is shown in side view the drive body 12 including the piston 13, which is shown somewhat occluded by the surrounding reservoir 11. Piston 13 may be a soft neoprene or similar FDA (United States Food and Drug Administration)-approved compound, such as is commonly used in the plunger of disposable syringes, which is fitted over a ridged end or bulbous nub at the end of the drive body so as to be controllably moveably thereby. The precise mode of attachment is well known in the art, and accordingly no particular detail of that structure is shown. The portion of the drive body 12 which is immediately behind the soft front face of the piston 13 is substantially circular in cross-section, tapering to a long narrow body, which is shaped substantially like a segment of a cylinder of roughly constant thickness, threaded on the inside. The threads 31 are shown in cross-section. The precise shape of the long cylindrical segment is not critical so long as the exterior approximately conforms to the inner contour of the reservoir and the segment is not unduly wide or narrow. For the long portion of the piston member, a width of between 1.59 and 9.53 mm (1/16 and 3/8 of an inch) has been found to be practical, as it provides good engagement with the drive gear 14, has adequate rigidity and compressive strength to transmit the drive force, and is thin enough to avoid causing frictionally induced motion from the turning of the drive screw gear 14.

Also shown in Figure 3 is the screw gear 14 attached to the shaft of motor 35. As shown in Figure 3 the motor has its principal cross-sectional dimension less than the diameter of the screw gear, so that it fits entirely within the cylindrical region formed by rotating the piston member 12 about the common central axis of the reservoir/piston assembly. An electromagnetic motor having an integral high-ratio gear reduction unit coaxially mounted in a common housing between the motor and drive gear has been used. This allows the motor 35 to drive the piston member 12 directly via the drive screw gear 14 without requiring other shafts, gears, pulleys, journals or other mechanical coupling or supporting elements in housing 16, resulting in a reduction of both inertial load and frictional losses as compared to conventional pumps. The screw gear drive may be of any appropriate pitch with the choice of pitch dependent on the cross-sectional area of the reservoir, the desired delivery rates and infusion pressures, the available motor speed and torque output and the desired operational cycles.

In constructing the screw drive, the thread profile of the screw gear in relation to the threads of the inner surface of the drive body is of some importance. In order to reduce backlash in the drive assembly, it is useful either to provide sharp peaks on the drive gear threads, so that the gear bites somewhat into the trenches of the threads of the drive body, or to provide a slight convexity or preloading in the face of the threads of either the drive gear or the drive body.

The operative metering principle involved in the control system of the present pump is that rotations of the drive screw 14 are directly proportional to the linear displacement of piston, hence to the volume of medicine delivered. The automatic control system for the present pump utilizes an internal light source which is reflected by a segmented optical disc connected to the drive train and detected by a photodetector so as to generate, in a manner known in the art, pulses representative of drive screw rotation. The control system operates the pump, starting at a predetermined time, and continuing until a predetermined number of pulses have been generated.

Because the reservoir 11 holds sufficient medication for many hours or days of therapy, extreme precautions must be taken to assure that the pumps do not have faults which might result in a continuous pumping failure mode. This might occur for instance if the light source failed to turn on, or burned out, so that no reflected pulses signalled the continued operation of the pump. To prevent the occurrence of such dangerous events, a novel arrangement is used in the drive system of the pump. Specifically the light and drive motor are wired in series or otherwise arranged so that both necessarily receive power if and only if the other is receiving and drawing power. In addition, condition sensing and power enabling circuitry are provided to further assure the device will shut down in the event a drive-sensing component fails. In particular a second photodetector is provided to detect direct (as opposed to reflected) light from the internal light source; in the event that the second photodetector fails to detect light from the internal light source, the circuitry causes power to be removed from light-drive motor combination. With this approach the motor is powered and medicine is delivered to the patient only if the motor, the light, and the turn-counting mechanism are all functioning. This circuit eliminates the dangers of undetected motor jamming or lamp burnout. This peculiar arrangement of the operating components with the logic and condition sensors necessarily assures that none of the permutations of component failure can result in a dangerous operating condition, but merely in shut-down of the device.

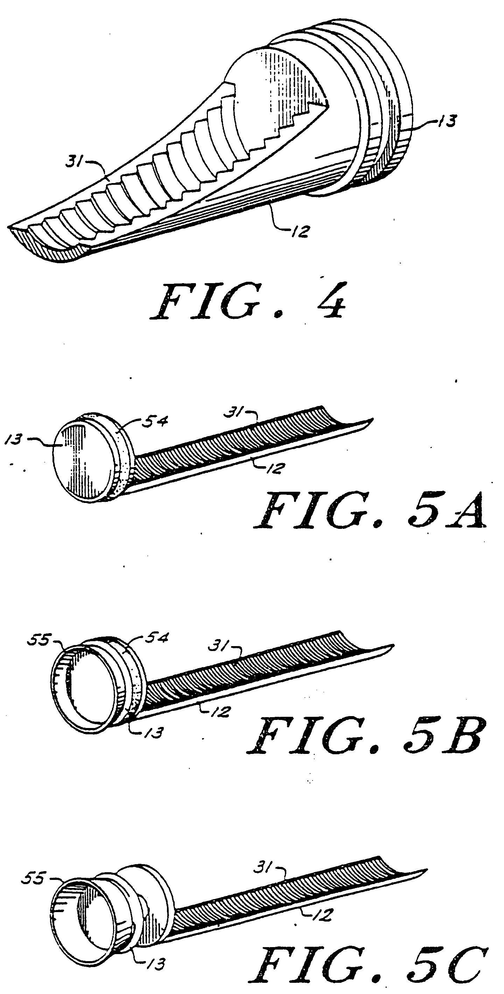

Turning now to Figure 4, there is shown the drive body 12 having internal threads 31 disposed along the length of the piston skirt. By engagement with the short rotating screw gear 14 the threads are operative to drive the piston a distance equal to the length of the threads 31. Because the piston skirt must slide laterally into engagement with the drive screw, the skirt comprises not much more, and preferably less, than a semi-circumferential portion of the cylindrical surface. In fact, since the piston skirt is used to transmit a linearly directed drive force, shear forces are negligible; and because the piston 13 is joined to the skirt portion along an arcuate edge, the skirt may safely be quite narrow.

Such a drive body 12, having a narrow skirt, is shown in Figures 5A, 5B and 5C, each of which shows an alternative embodiment of the piston face. In Figure 5A there is shown a drive body having a piston 13 in which a circumferential groove holds a sealing ring 54. In Figure 5B a variation of the piston head with sealing ring 54 is shown, further having a flared cup 55. The flared cup provides a strong seal against fluid leakage in the direction opposing the flare. In Figure 5C is shown a piston member having an integrally-formed head with a frontal flared cup 55, and a spaced-apart rear portion 56, which, because of its thinness, may be of a large diameter and operate as a bidirectional seal and scraper. The piston skirt in any of these embodiments preferably has one or more longitudinal ridges, 122 in Figure 2, arrayed along its exterior surface, to minimize the area of contact with the reservoir wall and to diminish frictional drag.

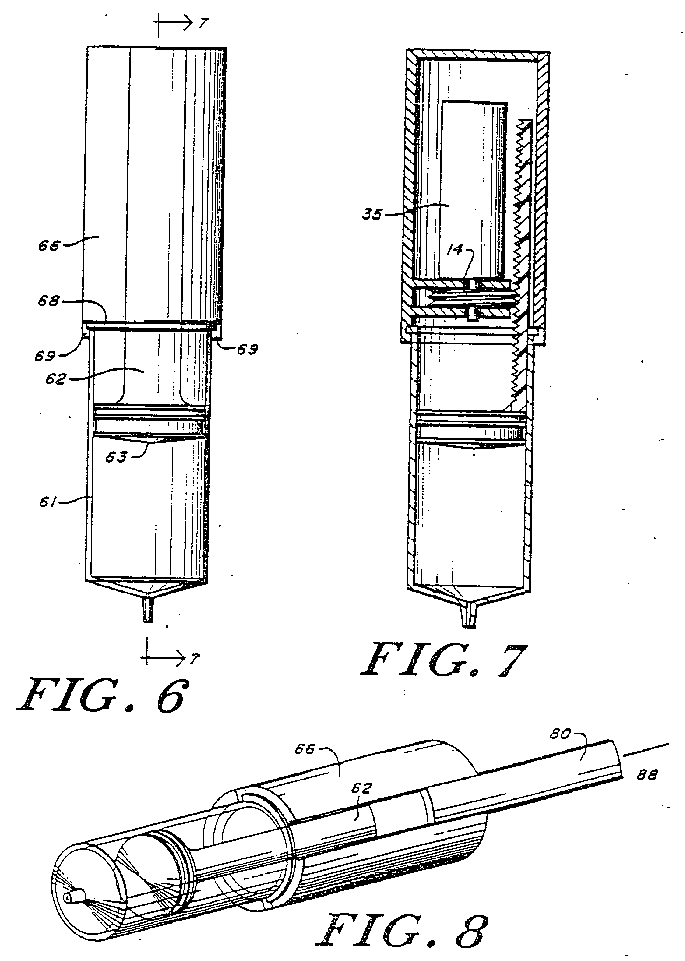

Turning now to Figure 6, there is shown an embodiment of the present invention usable as a large volume or macro-pump which may be suspended for bedside use in a vertical orientation in a manner similar to a conventional gravity infusion reservoir. The reservoir and drive assembly of the macro-pump are scaled-up versions of the device of Figure 1, having a housing 66 which accepts a reservoir 61 having a drive body 62. The reservoir is removably attached to the housing as by lip 68 of the reservoir and circumferential groove 69 in the housing, or by a breech lock or similar arrangement. The drive body 62 includes a piston face 63, which may be further sealed using an O-ring or any of the structural variations shown in Figures 5A, B or C.

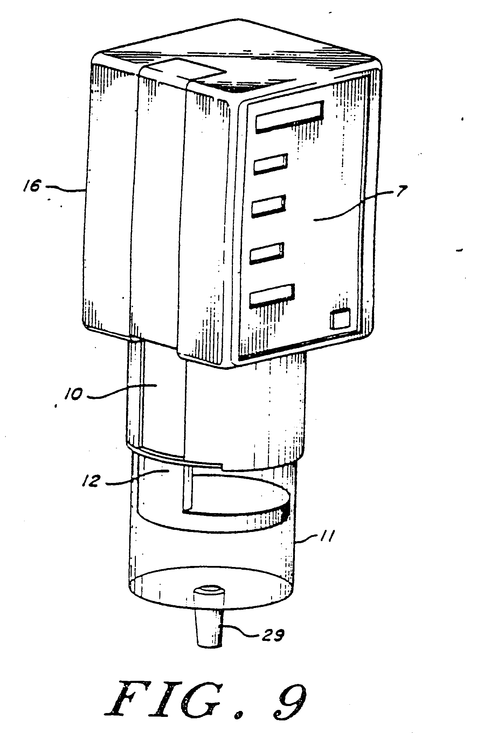

In Figure 7 is shown a cutaway view along line 7-7 of the embodiment of Figure 6, in which a motor 35 and drive screw 14 are shown driveably engaging internal threads of drive body 62. Figure 8 further shows a slot in housing 66 for holding drive body 62 in alignment. After insertion of the reservoir assembly into the housing a sliding plate 80 may be moved along axis 88 to close the slot and hold the piston drive body 62 in engagement with screw gear 14. Alternate means for biasing the drive assembly are possible. Furthermore, while Figure 7 shows a drive shaft journalled in the housing, such housing journals are not necessary, and the drive screw assembly may be simply mounted on the motor shaft, and the motor attached to the housing.

Because of the rigidity of the reservoir and the precise volumetric relationship between drive rotations and piston displacement the macro-pump embodiment is readily adaptable to microprocessor drive control and information display in a manner to yield extremely precise dosage control. This rigid piston and reservoir configuration eliminates the air space characteristic of bottle reservoirs (which consequently require venting, drip chambers, and other equipment) and the undefined volume characteristic of bag reservoirs (which do not permit ready indication delivered fluid volume), achieving in a single apparatus the precision normally associated with separate, expensive, dosing pumps. The housing 66 may house both a microprocessor controller and a battery power supply.

Figure 9 shows such a device, in which the housing 16 includes a keyboard 7 on one face, comprising a small number of programming keys. The panel may incorporate a display similar to 9 of Figure 1, of a kind known in the art, on which may appear messages to guide an attendant in entering the program data related to reservoir volume, total dose and timing. Such a display may also display relevant information such as the total delivered dose, or time interval since last infusion, all of which may be conveniently coded, stored and retrieved starting with the basic operating pulse data of the pump drive and the coded program data of the microprocessor control, by techniques known in the art.

It should be noted that although the foregoing discussion speaks of the fabrication of one embodiment using a motor and reducing gear, it is by no means necessary that the motor be an electromagnetic rotary motor capable of continuous operation. Indeed, a stepping motor, or even a solenoid or piezo ratchet driven motor would be equally serviceable, as long as the device were capable of having its total angular rotation precisely controlled or monitored by the control means. Whereas a continuous duty rotary motor may prove best for conveniently infusing an initial slug of medication, a stepping motor or ratchet drive may prove the most efficient or reliable for sustained infusion of maintenance doses at very low rates. In that event, a solenoid-actuated ratchet gear and corresponding digital control signalling means may be provided in the space shown for the motor. Furthermore, while the drive body has been shown as having a long thin body for simplicity, the relevant structural limitation is that the drive body comprise not much more than about 180° of arc of a cylindrical surface so that it may be conveniently placed in engagement around the drive gear. Thus a plurality of suitably spaced elements with internal threads in registry, or a wider drive body could be employed instead of the single thin piston body shown.

Accordingly, while the invention has been described with reference to specific embodiments thereof, it is to be understood that the invention may be embodied in other forms without departing from the scope of the invention as defined by the following claims.

## Claims

1. A reservoir assembly for removable engagement with a motor drive, comprising:
(A) a reservoir (11); and
(b) a piston (13) having (i) a fluid contacting head portion located within the reservoir (11) and (ii) a drive body (12) for engagement with a motor drive and having an aperture (121) therein;
characterized by a severable stem (131), removably attaching the fluid contacting head portion to the drive body, protruding from the fluid contacting head portion through the aperture (121) in the drive body sufficiently far to prevent the drive body (12) from being engaged with the motor drive without the stem (131) first having been severed, so that when the piston (13) is placed in engagement with the motor drive the head portion is detached from the body and retraction of the head portion and refilling of the reservoir (11) are prevented.

2. A reservoir assembly according to claim 1 , characterized in that the stem (131) extends along a central longitudinal piston axis and blocks the motor drive from engaging an internal surface of the piston (13).

3. A reservoir assembly according to claim 1 or 2, characterized in that the stem (131) is of sufficient strength to transmit push and pull forces for filling and bleeding the assembly, but has a weak portion (132) which may be severed by twisting, so as to detach the head portion from the body.

4. A reservoir assembly according to claim 1, 2 or 3, characterized in that the stem (131) is integrally formed with the piston head portion.

5. A reservoir assembly according to any one of claims 1 to 4, characterized in that the drive body has affixed thereto a piston follower (12), threaded along its interior for a length greater than the desired total piston displacement.

## Patentansprüche

1. Eine Behälteranordnung für entfernbaren Eingriff mit einem Motorantrieb, umfassend:
(A) einen Behälter (11); und
(B) einen Kolben (13), der (i) einen sich innerhalb des Behälters (11) befindenden, Fluid kontaktierenden Kopfteil und (ii) einen Antriebskörper (12) zum Eingriff mit einem Motorantrieb und eine Öffnung (121) darin habend hat;
**gekennzeichnet** durch einen abtrennbaren Schaft (131), der den Fluid kontaktierenden Kopfteil an dem Antriebskörper entfernbar anbringt, von dem Fluid kontaktierenden Kopfteil durch die Öffnung (121) in dem Antriebskörper genügend weit vorsteht, um zu verhindern, daß der Antriebskörper (12) mit dem Motorantrieb in Eingriff gebracht wird, ohne daß der Schaft (131) zuerst abgetrennt worden ist, so daß, wenn der Kolben (13) in Eingriff mit dem Motorantrieb plaziert wird, der Kopfteil von dem Körper gelöst wird und das Zurückziehen des Kopfteils und das Wiederfüllen des Behälters (11) verhindert werden.

2. Eine Behälteranordnung gemäß Anspruch 1, dadurch **gekennzeichnet**, daß sich der Schaft (131) entlang einer Längsmittelkolbenachse erstreckt und den Motorantrieb gegen einen Eingriff mit einer inneren Oberfläche des Kolbens (13) blockiert.

3. Eine Behälteranordnung gemäß Anspruch 1 oder 2, dadurch **gekennzeichnet**, daß der Schaft (131) von genügender Festigkeit ist, um Druck- und Zugkräfte zum Füllen und Entleeren der Anordnung zu übertragen, aber einen schwachen Teil (132) hat, welcher durch Verwinden bzw. Verbiegen abgetrennt werden kann, so daß der Kopfteil von dem Körper gelöst wird.

4. Eine Behälteranordnung gemäß Anspruch 1, 2 oder 3, dadurch **gekennzeichnet**, daß der Schaft (131) integral mit dem Kolbenkopfteil ausgebildet ist.

5. Eine Behälteranordnung gemäß irgendeinem der Ansprüche 1 bis 4, dadurch **gekennzeichnet**, daß an dem Antriebskörper ein Kolbenfolger (12) befestigt ist, der entlang seinem Inneren über eine Länge, die größer als die gewünschte gesamte Kolbenverlagerung ist, mit Gewinde versehen ist.

## Revendications

1. Ensemble réservoir destiné à être connecté de manière détachable à un entraînement par moteur, comprenant :
(A) un réservoir (11) ; et
(b) un piston (13) ayant (i) une partie tête contactant le fluide situé à l'intérieur du réservoir (11) et (ii) un corps d'entraînement (12) pour venir en prise avec un entraînement par moteur et comportant une ouverture (121);
caractérisé par une tige sectionnable (131), reliant de manière détachable la partie tête contactant le fluide avec le corps d'entraînement, dépassant à partir de la partie tête contactant le fluide par l'ouverture (121) dans le corps d'entraînement, suffisamment loin pour empêcher le corps d'entraînement (12) de venir en prise avec l'entraînement par moteur sans que la tige (131) ait d'abord été sectionnée, de telle manière que, lorsque le piston (13) est mis en prise avec l'entraînement par moteur, la partie tête soit détachée du corps et que le retrait de la partie tête et le remplissage du réservoir (11) soient empêchés.

2. Ensemble réservoir selon la revendication 1, caractérisé en ce que la tige (131) s'étend selon un axis de piston longitudinal central et empêche l'entraînement par moteur de venir en prise avec une surface interne du piston (13).

3. Ensemble réservoir selon la revendication 1 ou 2, caractérisé en ce que la tige (131) a une résistance suffisante pour transmettre des forces de traction et de poussée pour remplir et vider l'ensemble, mais en ce qu'elle a une partie faible (132) qui peut être sectionnée par torsion, de manière à séparer la partie tête et le corps.

4. Ensemble réservoir selon la revendication 1, 2 ou 3, caractérisé en ce que la tige (131) est formée d'un seul tenant avec la partie tête du piston.

5. Ensemble réservoir selon l'une quelconque des revendications 1 à 4, caractérisé en ce que le corps d'entraînement comporte, fixé sur lui, un poussoir de piston (12), fileté le long de l'intérieur sur une longueur supérieure au déplacement total désiré du piston.
